# EUROPEAN PATENT APPLICATION

(11) **EP 4 663 727 A1**
(43) Date of publication of application: **17.12.2025**
(21) Application number: 25165956.1
(22) Date of filing: 25.03.2025
(51) Int. Cl.: C11B 13/00, C07C 67/00, C10L 1/02, C10L 1/28, C11C 3/04

(54) **PROCESS OF TRANSESTERIFICATION OF OILS HAVING BIOLOGICAL ORIGIN**

(30) Priority: 13.06.2024 IT 202400013507
(71) Applicant: Itelyum Regeneration S.p.A., 26854 Pieve Fissiraga (LO) (IT)
(72) Inventor: MAGNONE, Giuseppe, 26854 PIEVE FISSIRAGA (Lodi) (IT); NEGRI, Francesco, 26854 PIEVE FISSIRAGA (Lodi) (IT); DI SERIO, Martino, 26854 PIEVE FISSIRAGA (Lodi) (IT); GALLO, Francesco, 26854 PIEVE FISSIRAGA (Lodi) (IT)
(74) Representative: Vatti, Francesco Paolo

(57) **Abstract**

A process of transesterification of oils having biological origin is disclosed, comprising the reaction of oils with alcohols in the presence of an acid or a base as a catalyst. According to the invention, the reaction occurs in the presence of an antifoaming agent, preferably a siloxane.

## Description

The present invention relates to a process for the transesterification of oils having biological origin, as well as to the use of siloxane agents as additives in a process for the transesterification of oils having biological origin.

The esterification reaction and its opposite, known as saponification or hydrolysis, are among the best-known reactions in organic chemistry. The esterification reaction involves the reaction of a carboxylic acid and an alcohol, in the presence of an acid or a base as a catalyst. The reverse reaction is called saponification if it occurs in an alkaline environment and, in this case, results in an alcohol and a salt of the organic acid. If, on the other hand, the hydrolysis reaction takes place in an acidic environment, alcohol and acid are formed.

Alongside these reactions, there is another one, called transesterification. Under conditions similar to those of the reactions seen above, an ester and an alcohol, that is different from the one that constitutes the starting ester, are reacted. In substance, there is hydrolysis of the ester and subsequent esterification of the resulting acid, with the added alcohol replacing the one that formed the starting ester, with the latter remaining in the reaction product.

The transesterification reaction is very useful, in particular, in the chemistry of fatty acids. Using this, esters can be given properties that are even very different from those of the starting ester. This reaction is widely used, for example, in the production of cosmetics.

The Applicant is active in the field of waste oil recovery. In recent years, the Applicant - starting from the recovery of mineral oils - has dedicated itself, in particular, also to the recovery and regeneration of used oils and fats, having biological origin, mainly having the nature of an ester. In order to obtain regenerated products that are useful for their intended purposes, it is often necessary to resort to a transesterification reaction. These processes are very important and often involve very large volumes. In particular, using NaOCH₃ as a catalyst, reactions of the following type occur:

ROH + Na⁺ + CH₃0⁻ ---> CH₃OH + RONa

RONa + R'COOH ---> R'COOR + Na⁺

Since the transesterification reaction proceeds passing through the hydrolysis of the starting ester, in particular, under alkaline conditions, the formation of salts of fatty acids, which are normally the same as the salts used as soaps, occurs. The formation of foams is therefore among the characteristics of these salts. In substance, given the volumes involved, there is a formation of not insignificant quantities of foam.

The formation of foam in these reactions is disadvantageous, since foams tend to expand, occupying large volumes and requiring equipment that is more voluminous than would be necessary if they were not present. Furthermore, they can pose risks of explosions, uncontrolled spills of material and partial removals of useful substances from the reaction vessels.

At present, this drawback is practically accepted as unavoidable by all those practicing transesterification, even if it causes a deterioration of the quality of the product and a reduction in the efficiency of the installations. Normally, foams are nowadays removed by alternating several washing steps, each followed by a corresponding centrifugation step. In this way, there are long reaction times and more expensive equipment is needed, and some of the useful product is lost.

The underlying problem of the invention is to propose a process for the transesterification of oils having biological origin, in particular, lubricants having biological origin, which process overcomes the mentioned drawbacks and enables a reaction under optimal conditions, with high efficiency and low operating costs. This purpose is achieved, according to a first aspect, through a process of transesterification of oils having biological origin, comprising the reaction of oils with alcohols in the presence of an acid or a base as a catalyst, characterised in that the reaction occurs in the presence of an antifoaming agent and in that said siloxane is added to the reactants in such a quantity as to constitute up to 1% by weight of the reaction mixture. According to a second aspect, the present invention relates to the use of siloxanes as additives in a process of transesterification of oils having biological origin. The dependent claims describe preferred features of the invention.

According to one embodiment, a siloxane is used as an antifoaming agent.

According to one embodiment, the siloxanes used have the general formula - (OSiR₁R₂)-ₙ wherein R₁ and R₂ are two alkyl groups, either the same or different from each other. Preferably, R₁ and R₂ are chosen from the group consisting of methyl, ethyl, propyl, and butyl.

According to one embodiment, R₁ and R₂ are both a methyl group.

According to one embodiment, the transesterification reaction takes place at a temperature comprised between 140 °C to 180 °C, with a pressure of 100-500 mbar, for a time of 4-8 hours.

According to one embodiment, oils having biological origin to be regenerated are used edible oils, specifically UCO (Used Cooking Oils) and RUCO (Regenerated Used Cooking Oils).

Additional features and advantages of the invention are, however, more apparent from the following detailed description of a preferred embodiment, provided solely by way of example and not limited thereto, and shown in the sole attached figure, which represents a vessel in which a transesterification reaction occurs in the absence of the present invention.

As mentioned, the present invention relates to a process for transesterification of oils having biological origin. Oils having biological origin are understood to be oils of animal and/or vegetable origin. As mentioned in the background portion, the reaction takes place in the presence of changeablequantities of the substitute alcohol; the actual quantity used depends mainly on the thermodynamics of the reaction.

Normally, the reaction takes place in the presence of a solvent and of an acidic or basic catalyst, which promotes both the hydrolysis reaction of the starting ester and the esterification reaction with the new alcohol. Preferably, the transesterification according to the invention is carried out at a temperature ranging between 140 °C and 180 °C, at a pressure of 100-500 mbar and for a time of 4-8 hours.

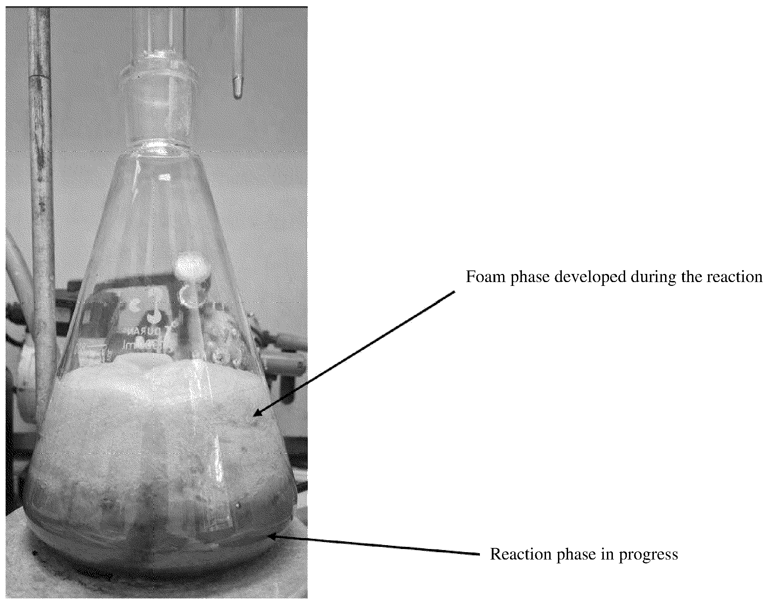

As can be seen from the attached figure, this reaction entails the formation of a considerable quantity of foam, which leads to the drawbacks seen in the preamble portion. Actually, the volume occupied by the foam is greater than that occupied by the actual reaction.

According to the present invention, an antifoaming agent is used to remedy this problem, although this has never been done in the past. Preferably, said antifoaming agent is a siloxane, which is readily available commercially and very effective. Siloxanes have a general formula -(OsiR₁R₂)ₙ. R₁ and R₂ can be the same or different and, preferably, are alkyl groups. Most preferably, R₁ and R₂ are chosen from the group consisting of: methyl, ethyl, propyl, and butyl. Methyl is particularly preferred, because a lower molecular weight of siloxane leads to better results in preventing the formation of foam. Therefore, the use of a siloxane wherein R₁ and R₂ are both a methyl group is particularly preferred, as it gives the absolute best results. The addition of said siloxane totally eliminates the formation of foam, and the contents of the vessel shown in the figure are reduced to just the bottom layer, with the complete elimination of the foam layer. This results in being able to employ a smaller volume of the vessels involved in the reaction, better heat management, the establishment of lower pressures, and complete recovery of the product, with a residual siloxane content in the product that, for practical purposes, is entirely insignificant. These advantages lead either to a simplification of the installations where the transesterification of these oils is carried out or to an increased productivity of the same installations, in any case with improved economic results.

The use of antifoaming agents had never been attempted before, because it was feared it would damage the product of the transesterification. It was found that the quantities sufficient to remove the foam - up to a maximum of 1% by weight - do not create any problems for the products, which remain sufficiently pure for their intended commercial and/or industrial purposes. In particular, this is true for the siloxanes, which can be used in such small quantities that they are insignificant in the obtained products.

Preferably, the siloxanes are added into the reagent mixture in such an quantity that they constitute up to 1% by weight, a quantity that, as mentioned above, is very limited and still achieves excellent results, despite the fact that the quantity is minimal.

According to the present invention, after the neutralisation of the reaction product, there is a clear separation of the latter from the aqueous phase, a fact that turns out to be extremely advantageous, in both technical and economical terms.

It is to be understood, however, that the invention is not to be considered limited to the particular arrangement illustrated above, which is only an exemplary embodiment of it, but rather that several variations are possible, all within the reach of a person skilled in the art, without hereby going beyond the scope of protection of the invention itself, as defined by the following claims.

## Claims

1. Process of transesterification of oils having biological origin, comprising the reaction of oils with alcohols in the presence of an acid or a base as a catalyst, **characterised in that** the reaction occurs in the presence of an antifoaming agent and **in that** said siloxane is added to the reactants in such an quantity as to constitute up to 1% by weight of the reaction mixture.

2. Process according to claim 1), **characterised in that** a siloxane is used as an antifoaming agent.

3. Process according to any one of the previous claims, **characterised in that** the siloxanes used have a general formula - (OSiR₁R₂)-ₙ wherein R₁ and R₂ are two alkyl groups, either the same or different from each other.

4. Process according to claim 3), **characterised in that** R₁ and R₂ are chosen from the group consisting of methyl, ethyl, propyl, and butyl.

5. Use of siloxanes as antifoaming agents in a process of transesterification of oils having biological origin, **characterised in that** said siloxane is added to the reactants in such a quantity as to constitute up to 1% by weight of the reaction mixture.

6. Use according to claim 5), **characterised in that** said siloxane has a general formula -(OSiR₁R₂)-ₙ, wherein R₁ and R₂ are two alkyl groups, either the same or different from each other.

7. Use according to claim 6), **characterised in that** R₁ and R₂ are chosen from the group consisting of methyl, ethyl, propyl, and butyl.

8. Use according to claim 7), **characterised in that** R₁ and R₂ are both a methyl group.
